# EUROPÄISCHE PATENTANMELDUNG

(11) **EP 3 530 753 A1**
(43) Veröffentlichungstag der Anmeldung: **28.08.2019**
(21) Anmeldenummer: 18195189.8
(22) Anmeldetag: 18.09.2018
(51) Int. Cl.: C12Q 1/6848

(54) **VERFAHREN ZUR PRIMER-VERLÄNGERUNGSREAKTION MIT VERBESSERTER SPEZIFITÄT**

(30) Priorität: 26.02.2018 EP 18158720
(71) Anmelder: AGCT GmbH, 23562 Luebeck (DE)
(72) Erfinder: Die Erfindernennung liegt noch nicht vor
(74) Vertreter: Schulz Junghans Patentanwälte PartGmbB

(57) **Zusammenfassung**

Die vorliegende Erfindung betrifft ein Verfahren zur Extension eines Primer-Oligonukleotids mit verbesserter Spezifität unter Verwendung eines besonderen Primer-Oligonukleotids und eines Controller-Nukleotids, wobei das Controller-Oligonukleotid in sequenzspezifischer Weise eine Strangöffnung in einem Doppelstrang aus Extensionsprodukt und Matrize ermöglicht. Die Erfindung betrifft weiterhin ein entsprechendes Kit zur Durchführung des erfindungsgemäßen Verfahrens.

## Beschreibung

Die vorliegende Erfindung betrifft ein Verfahren zur matrizenabhängigen Primer-Verlängerungsreaktion mittels einer matrizenabhängigen Polymerase. Bei dem Verfahren können Primer-Verlängerungsprodukte hergestellt werden, welche in einer Amplifikation von Nukleinsäuren eingesetzt werden können. Das Verfahren kann somit zur Verbesserung der Spezifität von Analysen umfassend einen Primer-Verlängerungs-Schritt beitragen.

### Hintergrund

Die Primerverlängerungsreaktion spielt bei sehr vielen genetischen Analysen eine Rolle, z.B. in Amplifikationsverfahren, wie der PCR. Es wird eine enzymatische Primerverlängerung mittels einer Polymerase durchgeführt, wobei ein synthetisierter Strang gebildet wird. In vielen Verfahren wird dieser Strang durch verschiedene Mittel sequenzunabhängig von der Matrize abgelöst und in weiteren Verfahrensschritten verwendet. Zu solchen Mitteln zählen beispielsweise Temperatur, denaturierende Agenzien wie Laugen oder Formamid. Weiterhin können Stränge mittels Enzymen unter Aufwendung von chemischen Energiequellen, wie dNTP oder ATP, getrennt werden. Dazu zählen Helicasen oder Polymerasen mit strangverdrängender Wirkung. Solche Trennungen erfolgen vorwiegend sequenzunspezifisch. Insbesondere spielt das Ergebnis eines korrekten Primings (Ausbildung eines Primer-Matrizen-Komplexes) als auch Fortführung der matrizenabhängigen enzymatischen Synthese mittels einer Polymerase eine Rolle. Mit der Erstellung des Produktes einer sequenzspezifischen Primerverlängerungsreaktion (Primerverlängerungsprodukt) ist die Synthese-Phase abgeschlossen. Nach Abschluss der Primerverlängerung kann das Primerverlängerungsprodukt von der Matrize abgelöst werden und in anderen Verfahrensschritten verwendet werden. In jedem dieser Schritte können Fehler oder Abweichungen stattfinden, welche die Spezifität der jeweiligen Verfahren beeinflussen können und schließlich auch bestimmte Grenzen der Einsetzbarkeit von jeweiligen Verfahren bedeuten. Viele Beispiele dafür sind bekannt, z.B. falsches Priming, polymerasebedingte Fehler, fehlerhafte Bindung von Sonden, etc.

Genetische Analyseverfahren müssen häufig unter mehreren Varianten einer bekannten Sequenz unterscheiden (z.B. Allel-spezifische Nachweismethoden oder Mutations-Analyse in Gegenwart von Wildtyp-Sequenzen). Die Anwesenheit von mehreren Sequenzen im gleichen Reaktionsansatz führt häufig dazu, dass es zu Interferenzen (ungewollten Interaktionen bzw. parallel laufenden Nebenreaktionen, etc.) zwischen den Sequenzen kommen kann. Solche Interferenzen können zu unterschiedlichen Zeiten eines Verfahrens entstehen, angefangen bei der Herstellung der ersten Kopien bis hin zur Detektion. Im Allgemeinen können Interferenzen zwischen sehr ähnlichen Sequenzen zu Fehlinterpretation von Ergebnissen führen. Eine hohe Spezifität bei solchen Verfahren spielt daher eine große Rolle.

Besonders bei geringfügigen genetischen Unterschieden, wie Einzelnukleotid-Veränderungen (SNV) und kurzen Insertionen/Deletionen (2 - 10 Nukleotide) wäre es von Vorteil, wenn die Spezifität von primerverlängerungsabhängigen Verfahren bzw. Verfahrensschritten verbessert werden kann.

Dabei kann sowohl die Synthese des Primerverlängerungsproduktes an sich als auch die Bereitstellung des synthetisierten Primer-Verlängerungsproduktes zu einer weiteren Verwendung verbessert werden.

Die Primerverlängerungsreaktion wird zur Zeit in den meisten Fällen durch Einstellung der Primerbindung (z.B. durch Verwendung optimaler Reaktionstemperaturen, Pufferbedingungen und Komplementarität der Primer zu einer Primerbindungsstelle) optimiert. Bis auf diese Phasen der Primerverlängerungsreaktion (Priming und *proofreading* durch die Polymerase) wird die Primerverlängerungsreaktion in den meisten Fällen gar nicht weiter auf ihre Qualität überprüft.

Es ist daher eine Aufgabe der vorliegenden Erfindung, Verfahren und Komponenten zur Verbesserung der Spezifität insbesondere in Verfahren bereitzustellen, welche eine enzymatische, matrizenabhängige Synthese bei einer Primerverlängerungsreaktion umfassen.

Diese Aufgaben werden durch die Gegenstände der unabhängigen Ansprüche erfüllt. Weitere vorteilhafte Ausgestaltungen der Erfindung sind in den abhängigen Ansprüchen und der folgenden Beschreibung aufgeführt.

### Beschreibung der Erfindung

Gemäß einem ersten Aspekt wird ein Verfahren zur Verlängerung (Extension) eines Primer-Oligonukleotids zur Verfügung gestellt, wobei
a) eine Probe, die ein Nukleinsäurepolymer umfassend die Zielsequenz M umfasst, wobei die Zielsequenz in 5'-3'-Orientierung die Sequenzabschnitte MS und MP umfasst und MP unmittelbar in 3' von MS angeordnet ist, mit folgenden Komponenten in Kontakt gebracht wird:
b) ein Oligonukleotidprimer P, welcher in 5'-3'-Orientierung die Sequenzabschnitte PC und PM umfasst und PM unmittelbar in 3' von PC angeordnet ist, wobei PM die zum Sequenzabschnitt MP komplementäre [die hybridisierende] Sequenz aufweist [im Wesentlichen sequenzspezifisch binden kann] und PC nicht an M [oder eine in 3' unmittelbar an MP anschließende Sequenz] binden kann;
c) ein Controller-Oligonukleotid C, welches in 5'-3'-Orientierung die Sequenzabschnitte CS, CP und CC umfasst, wobei CP unmittelbar in 3' von CS und unmittelbar in 5' von CC angeordnet ist, und wobei CS identisch mit MS ist und die Nukleinsäuresequenz von CP identisch zur Nukleinsäuresequenz von MP ist und CC die zum Sequenzabschnitt PC komplementäre [die hybridisierende] Sequenz aufweist und wobei CS modifizierte Nukleotidbausteine umfasst, so dass CS nicht als Matrize für die Aktivität der matrizenabhängigen Nukleinsäurepolymerase dienen kann;
d) eine matrizenabhängige Nukleinsäurepolymerase, insbesondere eine DNA-Polymerase, sowie Substrate der matrizenabhängigen Nukleinsäurepolymerase (insbesondere Ribonukleosid-triphosphate oder Desoxyribonukleosid-triphosphate) und geeignete Kofaktoren,
wobei ein Primer-Verlängerungsprodukt P' erhalten wird, insbesondere als Ergebnis einer matrizenabhängigen Verlängerung des Primers P durch die matrizenabhängige Polymerase, welches neben den Sequenzbereichen PC und PM einen synthetisierten Bereich PS umfasst, der im Wesentlichen komplementär zur Zielsequenz MS ist,
und wobei
- entweder die Reaktionsbedingungen so gewählt sind, und/oder
- die Länge und Schmelztemperatur von PC und/oder MS so gewählt sind,
dass P' mit M einen Doppelstrang bilden kann und P' bzw. ein Teil von P mit C einen Doppelstrang bilden kann und die Bildung des Doppelstranges aus P' und C gegenüber der Bildung des Doppelstrangs aus P' mit M bevorzugt ist, bzw. zur Trennung P' und M führen kann..

Insbesondere ist eine Nukleinsäuresequenz am 3'Ende von CS mit einer Länge von mindestens 10 Nukleotiden, insbesondere die gesamte Nukleinsäuresequenz von CS, identisch zu einer Nukleinsäuresequenz am 3'Ende von MS.

In einer Ausführungsform des erfindungsgemäßen Verfahrens ist vorgesehen, dass der Sequenzabschnitt CS an seinem 3' Ende unmittelbar 5' des Sequenzabschnitts CP einen Sequenzabschnitt CS' von 5 bis 15 Nukleotidpositionen Länge enthält, welcher aus Nukleinsäureanaloga, insbesondere aus 2'O-Alkylribonucleotiden, besteht.

In einer weiteren Ausführungsform der Erfindung ist vorgesehen, dass M einen Sequenzabschnitt MB unmittelbar in 5' von MS umfasst, und wobei weiterhin ein Block-Oligonukleotid B, das an MB hybridisieren kann, mit der Probe in Kontakt gebracht wird, und wobei
a. B Nukleosidanaloga umfasst, die so ausgewählt sind, dass ein Hybrid von B mit MB die Reaktion der matrizenabhängigen Nukleinsäurepolymerase auf MB nicht erlaubt oder
b. die matrizenabhängige Nukleinsäurepolymerase so ausgewählt ist, dass ein Hybrid von B mit MB [auch wenn B aus natürlichen Nukleosiden oder Deoxynukleosiden besteht] die Reaktion der matrizenabhängigen Nukleinsäurepolymerase auf MB nicht erlaubt.

In einer weiteren Ausführungsform des erfindungsgemäßen Verfahrens ist vorgesehen, dass B mit der Probe in Kontakt gebracht wird, bevor die matrizenabhängige Nukleinsäurepolymerase mit der Probe in Kontakt gebracht wird.

In einer weiteren Ausführungsform des erfindungsgemäßen Verfahrens ist vorgesehen, dass das Verfahren im Wesentlichen isothermal durchgeführt wird.

Der Begriff "im Wesentlichen isothermal" bedeutet im Sinne der vorliegenden Beschreibung insbesondere, dass die Reaktionstemperatur innerhalb eines Temperaturbereichs von 10k Temperaturdifferenz zwischen der höchsten und der niedrigsten Temperatur durchgeführt wird.

In einer weiteren Ausführungsform des erfindungsgemäßen Verfahrens ist vorgesehen, dass die Probe nicht mit einem Primer-Oligonukleotid in Kontakt gebracht wird (oder dieses umfasst), welches an einen in PS gelegenen Sequenzabschnitt binden und von dem aus durch die matrizenabhängige Nukleinsäurepolymerase eine Synthese eines Gegenstrangs zu PS erfolgen kann (mit anderen Worten: das Verfahren führt nicht zur exponentiellen Amplifikation von M).

In einer weiteren Ausführungsform des erfindungsgemäßen Verfahrens ist vorgesehen, dass MS eine Länge von 20 Nukleotiden bis 200 Nukleotiden hat.

In einer weiteren Ausführungsform des erfindungsgemäßen Verfahrens ist vorgesehen, dass P eine Länge im Bereich von 15 Nukleotiden bis 100 Nukleotiden hat.

In einer weiteren Ausführungsform des erfindungsgemäßen Verfahrens ist vorgesehen, dass PC eine Länge im Bereich von 5 Nukleotiden bis 85 Nukleotiden hat, insbesondere dass PC zwischen 50% und 300% der Länge des Sequenzabschnitts PM hat.

In einer weiteren Ausführungsform des erfindungsgemäßen Verfahrens ist vorgesehen, dass C eine Länge im Bereich von 20 Nukleotiden bis 100 Nukleotiden hat.

In einer weiteren Ausführungsform des erfindungsgemäßen Verfahrens ist vorgesehen, dass die Probe eine Variante VM der Zielsequenz M umfasst, welche ≥85%, insbesondere ≥95%, ≥92%, ≥94%, ≥96% oder sogar ≥98% identisch zu M ist, wobei ein Abschnitt VMS der Variante dem Abschnitt MS hoch identitisch ist, und ein Abschnitt VMP dem Abschnitt MP hochidentisch ist, und VM in mindestens einer Position bzw. Sequenzabschnitt VMM von der Sequenz M verschieden ist, insbesondere eine oder mehrere Substitution(en), Insertion(en) und/oder Deletion(en) aufweist.

In einer weiteren Ausführungsform des erfindungsgemäßen Verfahrens ist vorgesehen, dass VMM eine Länge von 1 bis 20 Nukleotiden, insbesondere von 1 bis 3 Nukleotiden, aufweist.

In einer weiteren Ausführungsform des erfindungsgemäßen Verfahrens ist vorgesehen, dass VMM in dem von VMS umfassten Sequenzabschnitt liegt.

In einer weiteren Ausführungsform des erfindungsgemäßen Verfahrens ist vorgesehen, dass die Abschnitte MP und VMP ≥99%, insbesondere 100%, identisch sind.

In einer weiteren Ausführungsform des erfindungsgemäßen Verfahrens ist vorgesehen, dass die Abschnitte MS und VMS ausserhalb des Abschnitts VMM ≥99%, insbesondere 100%, identisch sind.

In einer weiteren Ausführungsform des erfindungsgemäßen Verfahrens ist vorgesehen, dass VMM < 30 Nukleotidpositionen, insbesondere <25, <15 oder <10 Nukleotidpositionen in 5' von VMP auf VM angeordnet ist.

In einer weiteren Ausführungsform des erfindungsgemäßen Verfahrens ist vorgesehen, dass weiterhin ein zweites Controller-Oligonukleotid VC mit der Probe in Kontakt gebracht wird, welches in 5'-3'-Orientierung die Sequenzabschnitte VCS, VCP und VCC umfasst, wobei VCP unmittelbar in 3' von VCS und unmittelbar in 5' von VCC angeordnet ist, und wobei VCS identisch mit VMS ist und VCP identisch mit MP ist und VCC die zum Sequenzabschnitt PC komplementäre [die hybridisierende] Sequenz aufweist und wobei VCS und/oder VCP modifizierte Nukleotidbausteine umfasst, so dass VCS nicht als Matrize für die Aktivität der matrizenabhängigen Nukleinsäurepolymerase dienen kann.

Insbesondere ist eine Nukleinsäuresequenz am 3'Ende von VCS mit einer Länge von mindestens 10 Nukleotiden, insbesondere die gesamte Nukleinsäuresequenz von VCS, identisch zu einer Nukleinsäuresequenz am 3'Ende von VMS.

In einer weiteren Ausführungsform des erfindungsgemäßen Verfahrens ist vorgesehen, dass VMM mindestens teilweise von dem von VMP umfassten Sequenzabschnitt umfasst wird.

In einer weiteren Ausführungsform des erfindungsgemäßen Verfahrens ist vorgesehen, dass die Abschnitte MS und VMS ≥95%, insbesondere ≥98%, ≥99% oder sogar 100% identisch sind.

In einer weiteren Ausführungsform des erfindungsgemäßen Verfahren ist vorgesehen, dass weiterhin ein zweiter Oligonukleotidprimer VP mit der Probe in Kontakt gebracht wird, welcher in 5'-3'-Orientierung die Sequenzabschnitte VPC und VPM umfasst und VPM unmittelbar in 3' von VPC angeordnet ist, wobei VPM die zum Sequenzabschnitt VMP komplementäre [die hybridisierende] Sequenz aufweist [im Wesentlichen sequenzspezifisch binden kann] und VPC nicht an VM [oder eine in 3' unmittelbar an VMP anschließende Sequenz] binden kann.

In einer weiteren Ausführungsform des erfindungsgemäßen Verfahrens ist vorgesehen, dass VMM am 5'-Ende von VMP liegt, mit anderen Worten, dass das 3'-Ende von VP bzw. VPM mit VMM hybridisiert.

In einer weiteren Ausführungsform des erfindungsgemäßen Verfahrens ist vorgesehen, dass weiterhin ein Kompetitor-Oligonukleotid KP mit der Probe in Kontakt gebracht wird, welches mit VMP hybridisiert bzw. hybridisieren kann, wobei insbesondere KP komplementäre Sequenzbereiche zu VMP umfasst.

In einer weiteren Ausführungsform des erfindungsgemäßen Verfahrens ist vorgesehen, dass die gewählten Reaktionsbedingungen eine Reaktionstemperatur im Bereich von 25 °C bis 80 °C umfassen.

Gemäß einem weiteren Aspekt der Erfindung wird ein Kit zur Durchführung des erfindungsgemäßen Verfahrens zur Verfügung gestellt. Das Kit umfasst:
- einen Oligonukleotidprimer P, welcher in 5'-3'-Orientierung die Sequenzabschnitte PC und PM umfasst und PM unmittelbar in 3' von PC angeordnet ist, wobei PM an eine Sequenz M, insbesondere eine genomische Sequenz, eines eukaryoten Organismus oder eines pathogenen Bakteriums, insbesondere eines Säugetiers, ganz inbesondere an eine menschliche (genomische) Zielsequenz, umfassend eine Primerbindungsstelle MP binden kann und PC nicht an eine direkt in 3' von MP liegende Sequenz binden kann; und
- einen Controller-Oligonukleotid C, welcher in 5'-3'-Orientierung die Sequenzabschnitte CS, CP und CC umfasst, wobei CP unmittelbar in 3' von CS und unmittelbar in 5' von CC angeordnet ist, und wobei CS identisch mit einer unmittelbar in 5' von MP liegenden Sequenz MS ist und CP identisch mit MP ist und CC die zum Sequenzabschnitt PC komplementäre [die hybridisierende] Sequenz aufweist, und wobei CS modifizierte Nukleotidbausteine umfasst, so dass CS nicht als Matrize für die Aktivität einer matrizenabhängigen Nukleinsäurepolymerase dienen kann.

Insbesondere ist eine Nukleinsäuresequenz am 3'Ende von CS mit einer Länge von mindestens 10 Nukleotiden, insbesondere die gesamte Nukleinsäuresequenz von CS, identisch zu einer Nukleinsäuresequenz am 3'Ende von MS.

In einer Ausführungsform des erfindungsgemäßen Kits ist vorgesehen, dass der Kit weiterhin Block-Oligonukleotid B umfasst, das an einen unmittelbar 5' von MS gelegenen Sequenzabschnitt der Zielsequenz MB hybridisieren kann, wobei B Nukleosidanaloga umfasst, die so ausgewählt sind, dass ein Hybrid von B mit MB die Reaktion einer matrizenabhängigen Nukleinsäurepolymerase auf MB nicht erlaubt.

In einer weiteren Ausführungsform des erfindungsgemäßen Kits ist vorgesehen, das der Kit weiterhin umfasst:
- einen zweiten Oligonukleotidprimer VP, welcher in 5'-3'-Orientierung die Sequenzabschnitte VPC und VPM umfasst und VPM unmittelbar in 3' von VPC angeordnet ist, wobei VPM an eine genomische Sequenz VM umfassend eine Primerbindungsstelle VMP binden kann, wobei VM zu M ≥85%, insbesondere ≥95%, ≥92%, ≥94%, ≥96% oder sogar ≥98% identisch zu M, aber in mindestens einer Position verschieden von M ist, und VPC nicht an eine direkt in 3' von VMP liegende Sequenz binden kann;
   und/oder
- ein zweites Controller-Oligonukleotid VC, welches in 5'-3'-Orientierung die Sequenzabschnitte VCS, VCP und VCC umfasst, wobei VCP unmittelbar in 3' von VCS und unmittelbar in 5' von VCC angeordnet ist, und wobei VCS identisch mit einer (unmittelbar in 5' von VMP liegenden) genomischen Sequenz VMS ist, die Teil einer Variante VM ist, die ≥85%, insbesondere ≥95%, ≥92%, ≥94%, ≥96% oder sogar ≥98% identisch zu M, aber in mindestens einer Position verschieden von M ist, und VCP identisch mit VMP ist und VCC die zum Sequenzabschnitt VPC komplementäre [die hybridisierende] Sequenz aufweist, und wobei VCS modifizierte Nukleotidbausteine umfasst, so dass VCS nicht als Matrize für die Aktivität einer matrizenabhängigen Nukleinsäurepolymerase dienen kann,
   und/oder
- ein Kompetitor-Oligonukleotid KP, welches mit VMP hybridisiert bzw. hybridisieren kann, insbesondere vorwiegend komplementär.

In einer weiteren Ausführungsform des erfindungsgemäßen Kits ist vorgesehen, dass der Kit eine matrizenabhängige Nukleinsäurepolymerase umfasst, insbesondere eine DNA-Polymerase, sowie optional Substrate der matrizenabhängigen Nukleinsäurepolymerase (insbesondere Ribonukleosid-triphosphate oder Desoxyribonukleosid-triphosphate) und geeignete Kofaktoren.

In einer weiteren Ausführungsform des erfindungsgemäßen Kits ist vorgesehen, dass dieser Kit **kein** Primer-Oligonukleotid umfasst, welches an einen auf dem Gegenstrang von M gelegenen Sequenzabschnitt binden und von dem aus durch die matrizenabhängige Nukleinsäurepolymerase eine Synthese einer im Wesentlichen zu dem 5' von MP auf M gelegenen Sequenz identischen Sequenz erfolgen kann (mit anderen Worten: der Kit ermöglicht nicht, die 5' von MP auf M gelegenen Sequenz exponentiell zu amplifizieren).

### Figurenbeschreibung

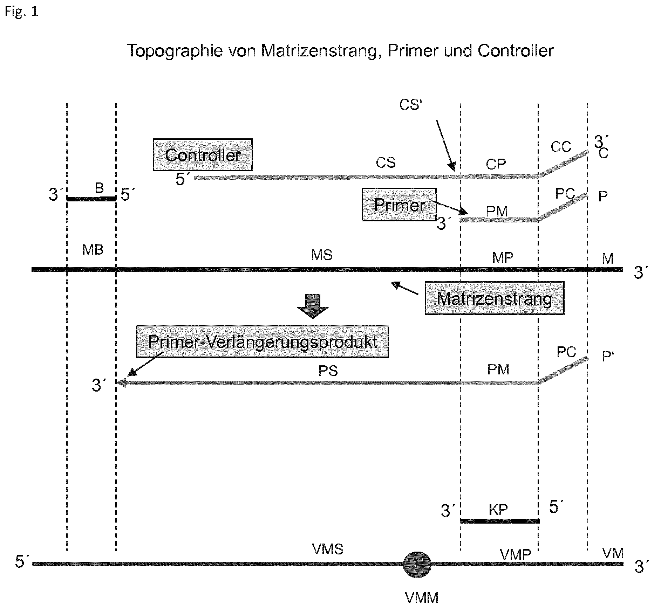

Fig. 1 zeigt schematisch eine Ausführungsform mit Topographie eines spezifischen Primer-Verlängerungs-Systems und eines Matrizenstrangs mit einem polymorphen Lokus (N2).
Fig. 2 zeigt Ergebnisse aus Beispiel 1.
Fig. 3 zeigt Ergebnisse aus Beispiel 2.
Fig. 4-6 zeigen Ergebnisse aus Beispiel 4.

### Beispiele

### Material und Methoden:

Amplifikationsreaktion Lösung 1: Kalium Glutamat, 50 mmol/l, pH 8,0; Magnesium Acetat, 10 mmol/l ; dNTP (dATP, dCTP, dTTP, dGTP), je 200 µmol/l; Polymerase (Bst 2.0 WarmStart, 120.000 U/ml NEB), 12 Units / 10 µl; Triton X-100, 0,1% (v/v); EDTA, 0,1 mmol/l; TPAC (Tetrapropylammonium Chloride), 50 mmol/l, pH 8,0; EvaGreen Farbstoff (Farbstoff wurde entsprechend Anleitung des Herstellers in Verdünnung 1:50 eingesetzt).

Amplifikationsreaktion Lösung 2: 1x Isothermal Puffer (New England Biolabs) dNTP (dATP, dCTP, dUTP, dGTP), je 200 µmol/l; EvaGreen Farbstoff (Farbstoff wurde entsprechend Anleitung des Herstellers in Verdünnung 1:50 eingesetzt).

### Beispiel 1:

### Primer-Verlängerungsreaktion zur Herstellung eines Primerverlängerungsproduktes unter Verwendung von Sequenzvarianten einer Zielsequenz und anschließende Verwendung des synthetisierten Primer-Verlängerungsproduktes in einer Amplifikationsreaktion.

In diesem Beispiel wurde der Einfluss von Sequenzunterschieden in zwei Matrizen auf die Interaktion mit dem Controller nach einer Synthese-Phase untersucht. Es wurden zwei Matrizen bereitgestellt, welche an einer Nukleotidposition eine Sequenzdifferenz umfassten. Bei Verlängerung des ersten Primer-Oligonukleotides wurde jeweils ein komplementärer Strang gebildet, welcher eine komplementäre Sequenz zur jeweiligen Matrize aufwies und somit ebenfalls diesen Sequenzunterschied in der Sequenz umfasste. Beide Matrizen umfassten eine einheitliche Primer-Bindungsstelle, so dass ein einheitlicher Primer verwendet werden konnte. Die Diskriminierung zwischen einzelnen Sequenzvarianten der Zielsequenz erfolgte somit mittels eines spezifischen Controller-Oligonukleoitides.

Die während der Synthese-Phase generierten Primer-Verlängerungsprodukte können durch den Controller vorwiegend spezifisch von Matrizensträngen abgelöst werden. Die Primer-Verlängerungsprodukte umfassen ein 3'-Segment, welches nicht von einem Controller-Oligonukleotid gebunden wird. Dieses 3'-Segment des ersten Primer-Verlängerungsproduktes kann von einem anderen Oligonukleotid, z.B. Primer, komplementär gebunden werden. Die auf diese Weise generierten ersten Primer-Verlängerungsprodukte können in einer anschließenden Amplifikationsreaktion unter Verwendung eines weiteren, zweiten Primers amplifiziert werden. Die Amplifikationsreaktion dient somit zur Veranschaulichung einer Auswirkung einer Controlling-Phase (Strang-Trennung durch einen Controller) nach einer Synthese-Phase.

Folgende Komponenten wurden verwendet:
- Matrize M2SF5-M001-200 (SEQ ID NO 1) mit einer Sequenz, welche zu einem ersten Primer-Verlängerungsprodukt mit einem Perfekt-Match Übereinstimmung mit Controller-Oligonukleotid führt;
- Matrize M2SF5-WT01-200 (SEQ ID NO 2) mit einer Sequenz, welche zu einem ersten Primer-Verlängerungsprodukt führt, das an einer einzelnen Basenposition ein Mismatch mit dem Controller-Oligonukleotid bildet;
- Primer-Oligonukleotid P1F5-200-AE2053 (SEQ ID NO:3);
- Controller-Oligonukleotid AD-F5-1001-503 (SEQ ID NO:4).

Die Primer-Verlängerungsreaktion wurde direkt als Amplifikationsreaktion fortgeführt (homogenes Assay). Zu diesem Zweck wurde vor Beginn der Primer-Verlängerungs-Reaktion ein weiterer Primer (Primer 2, P2G3-5270-7063) in das Reaktionsgemisch gegeben. Dieser zweite Primer kann an das 3'-Segment des in der Primer-Verlängerungsreaktion synthetisierten Primer-Verlängerungsproduktes komplementär binden. Weiterhin wird dieser zweite Primer genutzt, um eine Amplifikationsreaktion zu unterstützen, ausgehend vom während der Synthese-Phase matrizenspezifisch synthetisierten ersten Primer-Verlängerungsprodukt, welches anschließend in der Controlling-Phase von seinem Matrizen-Strang durch den Controller abgelöst wird. Das Primer-Segment ist in der Lage, an das 3'-Segment des ersten Primer-Verlängerungsproduktes komplementär zu binden und unter Verwendung des ersten Primer-Verlängerungsproduktes als Matrize eine zweite Primer-Verlängerungsreaktion zu generieren. Aus der ersten Primer-Verlängerungs-Reaktion und der zweiten Primer-Verlängerungsreaktion resultiert dabei ein Doppelstrang, umfassend das erste und das zweite Primer-Verlängerungsprodukt. Bei der weiterer Verwendung der beiden Primer und des Controller-Oligonukleotides (und wiederholten Temperaturen von 55°C und 65°C) ergibt sich eine exponentielle Amplifikation, welche sowohl Primer-Verlängerungsschritte als auch Strangtrennung mittels Controller-Oligonukleotid umfasst. Während der Amplifikations-Phase diente das erste Primer-Oligonukleotid weiterhin als Amplifikationsprimer.

Es wurden vier Ansätze vorbereitet:
Ansatz 1 enthält als Matrize M2SF5-M001-200 (Perfect Match Situation) in Konzentration von 300 fmol/l (entspricht ca. 2x10^6 Kopien/ Ansatz).
Ansatz 2 enthält die Matrize M2SF5-M001-200 (Perfect Match Situation) in Konzentration von 300 amol/l (entspricht ca. 2x10^3 Kopien/ Ansatz).
Ansatz 3 enthält keine Matrize und bildet somit eine Kontrolle.
Ansatz 4 enthält als die Matrize M2SF5-WT01-200 (single Mismatch Situation) in 300 pmol/l Konzentration (ca. 2x 10^9 Kopien pro Ansatz).

Primer 1 wurde mit 5 pmol/l, das Controller-Oligonukleotid mit 2 µmol/l und Primer 2 mit 1 µmol/l eingesetzt. Die Reaktionen wurden in Amplifikations-Lösung 2 durchgeführt. Um die Anwesenheit von genomischer DNA im Assay nachzustellen wurden pro Reaktion 100 ng frisch denaturierte Fisch-DNA (Lachs-DNA) zugegeben.

### Eine Primer-Verlängerungs-Reaktion (Synthese-Phase und Controlling-Phase).

Die thermischen Reaktionsbedingungen waren 2 min bei 55 °C (Synthese-Phase) und anschließend 5 min bei 65 °C (Controlling-Phase). Die Synthese-Phase der Primer-Verlängerungsreaktion des ersten Primers fand in erster Linie bei 55°C statt. Bei dieser Temperatur lag der Controller in Komplex mit dem ersten Primer vor und war damit in einem nicht-aktiven Zustand. Die Controlling-Phase fand bei 65°C statt. Bei dieser Temperatur dissoziierte der Controller zumindest teilweise vom ersten Primer-Oligonukleotid und konnte somit mit gebildeten Primer-Verlängerungsprodukten interagieren. Die Schmelztemperatur des ersten Bereichs des ersten Primers mit seiner Primer-Bindungsstelle an der Matrize betrug ca. 45°C in Amplifikations-Lösung 2. Somit wurde die Synthese-Phase bei einer Reaktions-Temperatur von etwa Tm +10°C durchgeführt, bezogen auf Tm von Matrize / erster Primer-Komplex. Aufgrund einer höheren Konzentration des ersten Primers (5 µmol) lag in der Synthese-Phase eine genügende Menge des ersten Primers frei, um an die Matrize zu binden und die Reaktion zu starten. Bei Erhöhung der Temperatur auf 65°C sank die Ausbeute der Synthese-Phase signifikant. Bei dieser Temperatur wurde der Controller aus seiner Bindung mit dem Primer teilweise freigesetzt (Tm des Komplexes umfassend erstes Primer und Controller in Amplifikations-Lösung 1 betrug 63°C). Daher verlief die Controlling Phase bei ca. Tm +2°C. bezogen auf Tm von Controller / erster Primer - Komplex).

### Amplifikations-Reaktion (Wiederholung von Primer-Verlängerungs-Reaktionen)

Die thermischen Reaktionsbedingungen waren zyklisch, alternierende Temperaturänderungen, wobei jeweils auf ein 2 min Zeitintervall bei 55 °C ein 5 min Zeitintervall bei 65 °C folgte. Die Amplifikation wurde über 100 Zyklen verfolgt. Detektion erfolgte bei 65°C für EvaGreen-Fluoreszenzsignal. Die erfolgreiche Synthese von Primer-Verlängerungs-Produkten (hier als Amplifikation bezeichnet) konnte durch einen Anstieg des EvaGreen-Fluoreszenzsignals im Laufe der Zeit festgestellt werden. Die TemperaturÄnderungen und Real-Time Monitoring wurde mit StepPne Plus Real-Time PCR Gerät von Thermofisher durchgeführt.

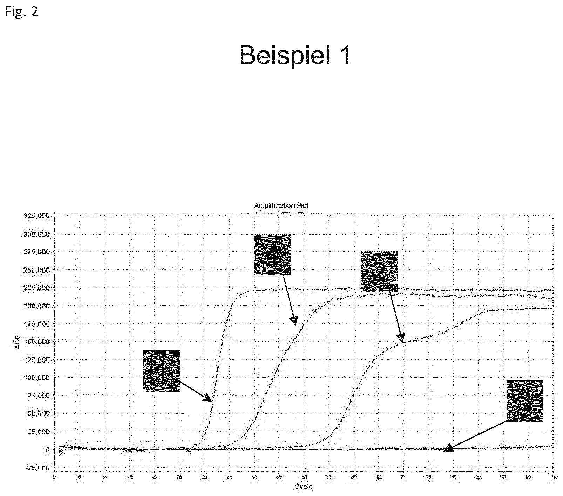

Fig. 2 zeigt einen typischen Verlauf des EvaGreen-Signals. Auf der Y-Achse ist die Zunahme des Fluoreszenzsignals (Delta Rn) und auf der X-Achse ist die Reaktionszeit (als Zyklusnummer) aufgetragen. Pfeile markieren einzelne Reaktionsansätze. Dabei gehören die markierten Positionen zu folgenden Ansätzen:

| | |
|---|---|
| Pfeil 1: 300 fmol/l perfekt-Match Matrize | (ca. 2x10^6 Kopien/Ansatz) |
| Pfeil 2: 300 amol/l perfekt-Match Matrize | (ca. 2x10^3 Kopien/Ansatz) |
| Pfeil 3: keine Matrize | |
| Pfeil 4: 300 pmol/l Mismatch Matrize | (ca. 2x 10^9 Kopien pro Ansatz). |

Man sieht den Anstieg des Fluoreszenz-Signals sowohl bei der perfekt-Match als auch bei der Mismatch-Variante der Matrize, wobei das Signal der Mismatch-Variante (4) trotz 1000fachen Überschußes später erscheint. Beim Single Mismatch wird eine Verzögerung von ca. 15 Zyklen beobachtet. Die zeitliche Verzögerung (=Zyklusanzahl) ist ein unmittelbares Maß für die Diskriminierung durch Wirkung von dem Controller. Bei Verwendung einer Perfect-Match Matrize kommt es zur Synthese eines komplementären Stranges eines Primer-Verlängerungsproduktes. Dieses Verlängerungsprodukt ist sowohl zur Perfect-Match-Matrize komplementär als auch zum verwendeten Controller-Oligonukleotid. Im Gegensatz dazu, unter Verwendung einer Mismatch-Sequenz kommt es bei der Synthese des ersten Primer-Verlängerungsproduktes zur Generierung eines komplementären Stranges des Verlängerungsproduktes, welches zwar eine vollständige Komplementarität zur Mismatch-Matrize aufweist, aber dadurch von der Komplementarität mit dem dritten Bereich des Controller-Oligonukleotids abweicht. Diese Abweichung findet im 5'-ständigen Segment des Verlängerungsproduktes statt, welches mit dem Controller-Oligonukleotid reagieren sollte, damit der Strangverdrängungsvorgang voranschreiten kann. Wie im vorliegenden Beispiel gezeigt wird, stört das Mismatch eine Strangtrennung durch das Controller-Oligonukleotid.

Dieses Ergebnis veranschaulicht die Bedeutung der Basenzusammensetzung im Controller-Oligonukleotid: Abweichungen von der Komplementarität zwischen Controller-Oligonukleotid und dem Primer-Verlängerungsprodukt können zu Verlangsamung oder sogar Unterbrechung der Amplifikation führen. In diesem Beispiel wurde gezeigt, dass obwohl Sequenz-Enden von Perfect-Match-Matrize und Mismatch-Matrize komplett übereinstimmten und somit das Potenzial zur Bindung von beiden Primer-Oligonukleotide gleich war, sind beide Reaktionen vollkommen unterschiedlich gelaufen: bei einer vollständigen Komplementarität zwischen dem Controller-Oligonukleotid und dem 5'-Segment des Verlängerungsproduktes des ersten Primer-Oligonukleotides verlief die Amplifikation planmäßig. Eine Unterbrechung der Strangtrennung während der Controlling-Phase durch eine nicht-komplementäre Sequenzvariante (in diesem Fall durch ein Mismatch) führte zur Minderung der Strang-Trennung, was sich in Unterdrückung der Amplifikations-Kinetik äußerte. Somit kann ein in einer Primer-Verlängerungs-Reaktion hergestelles Primer-Verlängerungsprodukt in einem Amplifikations-Verfahren als Start-Nukleinsäure verwendet werden.

### Beispiel 2:

### Einfluss eines Mismatches zwischen einem ersten Primer und einem Controller auf Strang-Trennung (Controlling-Phase).

Ähnlich wie im ersten Beispiel wurde eine Amplifikationsreaktion zur Veranschaulichung einer Auswirkung einer Controlling-Phase (Strang-Trennung durch einen Controller) nach einer Synthese-Phase verwendet.

Die Synthese-Phase findet unter Reaktionsbedinungen statt, welche eine Primer-Verlängerungsreaktion an einer Matrize ermöglichen. Zur jeweiligen Strangtrennung (Controller-Phase) wurden allerdings verschiedene Controller verwendet: Perfekt-Match- und Mismatch-Controller-Oligonukleotid zum verwendeten Primer während der Synthese-Phase. Um den Effekt eines solchen einzelnen Mismatches zwischen einem ersten Primer und einem Controller zu demonstrieren, wurden wiederholt Primer-Verlängerungsreaktionen (Synthese-Phasen) und Controlling-Phasen unter zyklischen Temperaturbedingungen durchgeführt. Es resultierte somit eine Amplifikation von einem ersten Primer-Verlängerungsprodukt.

Die Amplifikation diente dabei als Detektions-Methode für die Controlling-Phase: nur wenn Strang-Trennung erfolgreich verlaufen kann und das zweite Primer-Oligonukleotid an das 3'-Segment des ersten Primer-Verlängerungsproduktes binden kann, findet eine Amplifikation statt. Aufgrund einer hohen Sensitivität dieser Methode (wegen exponentiellen Charakters) und einer Quantifizierungs-Möglichkeit (Zeitabhänigkeit der Signaldetektion je nach Konzentration) wurde die Amplifikation als Detektions-Methode für eine erfolgreiche bzw. nicht erfolgreiche Strang-Trennung während einer Controlling Phase verwendet. Das Real-Time-Monitoring der Synthese von entstehenden Amplifikationsprodukten erfolgte in diesem Beispiel mit dem interkalierenden Farbstoff Eva Green.

Folgende Komponenten wurde verwendet:
- Matrize M2SF5-M001-200 (SEQ ID NO 1) mit Sequenzzusammensetzung, welche zu einem ersten Primer-Verlängerungsprodukt mit einer Perfekt-Match-Übereinstimmung mit dem Controller-Oligonukleotid führt:
- erstes Primer-Oligonukleotid P1F5-200-AE2053 (SEQ ID NO 3) als Primer-Verlängerungsprimer und als Amplifikations-Primer;zweites Primer-Oligonukleotid P2G3-5270-7063 (SEQ ID NO 5 oder 6) als zweiter Amplifikations-Primer;
- Perfekt-Match-Controller-Oligonukleotid AD-F5-1001-503 (SEQ ID NO 4)
- Mismatch-Controller-Oligonukleotid MD2AD-F5-011-5 (SEQ ID NO 7)

Sowohl der erste Primer als auch der zweiter Primer sind hierbei in der Lage, eine Amplifikation mit einem perfekt-match Controller-Oligonukleotid zu unterstützen. Im Mismatch Controller-Oligonukleotid wurde in der Position -24 (vom 3'-Ende) ein A-Nukleotid (Mismatch-Variante) anstatt eines G-Nukleotides (perfekt Match Variante) eingesetzt. Diese Position bildet ein Mismatch mit dem 3'-terminalen Nukleotid des ersten Primers. Die Matrize wurde in Konzentrationen von 1-3 pmol/l eingesetzt. In der Kontroll-Reaktion wurde keine Matrize eingesetzt (= Negativkontrolle). Primer 1 wurde mit 5 µmol/l, das jeweilige Controller-Oligonukleotid mit 2 µmol/l und Primer 2 mit 1 µmol/l eingesetzt. Die Reaktion wurden in Amplifikations-Lösung 2 durchgeführt. Um die Anwesenheit von genomischer DNA im Assay nachzustellen wurden pro Reaktion 100 ng frisch denaturierte Fisch-DNA (Lachs-DNA) zugegeben.

Die Synthesephase der Primer-Verlängerungs-Reaktion des ersten Primers fand in erster Linie bei 55°C statt. Bei dieser Temperatur lag der Controller in Komplex mit dem ersten Primer und war damit in einem nicht-aktiven Zustand. Die Controlling-Phase fand bei 65°C statt. Bei dieser Temperatur dissoziierte der Controller zumindest teilweise vom ersten Primer-Oligonukleotid und konnte somit mit gebildeten Primer-Verlängerungsprodukten interagieren. Die Schmelztemperatur des ersten Bereichs des ersten Primers mit seiner Primer-Bindungsstelle an der Matrize betrug ca. 45°C in Amplifikations-Lösung 2. Somit wurde die Synthese-Phase bei einer Reaktions-Temperatur von etwa Tm +10°C durchgeführt, bezogen auf Tm von Matrize / erster Primer-Komplex. Aufgrund einer höheren Konzentration des ersten Primers (5 µmol) lag in der Synthese-Phase eine genügende Menge des ersten Primers frei, um an die Matrize zu binden und die Reaktion zu starten. Bei Erhöhung der Temperatur auf 65°C sank die Ausbeute der Synthese-Phase signifikant. Bei dieser Temperatur wurde der Controller aus seiner Bindung mit dem Primer teilweise freigesetzt (Tm des Komplexes umfassend erstes Primer und Controller in Amplifikations-Lösung 1 betrug 63°C). Daher verlief die Controlling Phase bei ca. Tm +2°C. bezogen auf Tm von Controller / erster Primer - Komplex. In der Amplifikation waren die thermischen Reaktionsbedingungen zyklisch, alternierende Temperaturänderungen, wobei jeweils auf ein 1 min Zeitintervall bei 55 °C ein 5 min Zeitintervall bei 65 °C folgte. Die Amplifikation wurde typischerweise über 120 Zyklen, d.h. 120 x (1 min 55°C + 5 min 65°C) = 120 x 6 min = 12 h verfolgt. Die erfolgreiche Amplifikation konnte durch einen Anstieg des EvaGreen-Fluoreszenzsignals im Laufe der Zeit festgestellt werden.

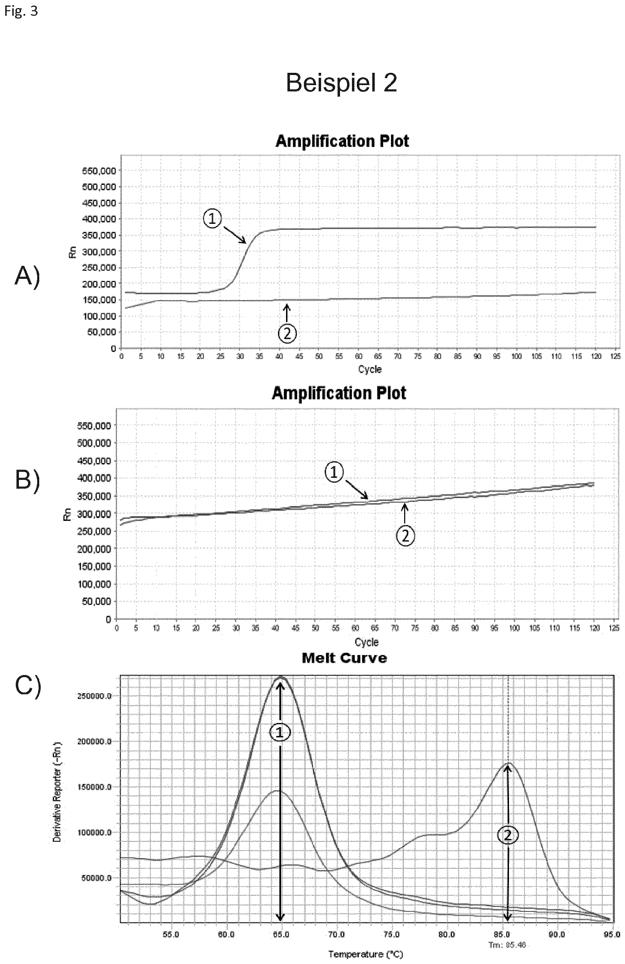

Fig. 3A zeigt einen typischen Verlauf des EvaGreen-Fluoreszenzsignals im Laufe einer Allelspezifischen Amplifikationsreaktion unter Verwendung eines Perfekt-Match-Controller-Oligonukleotids. Auf der Y-Achse ist das Fluoreszenzsignal des EvaGreen-Farbstoffes und auf der X-Achse ist die Reaktionszeit (als Zyklusnummer) aufgetragen. Pfeil 1 markiert in Fig. 3A einen Perfekt-Match-Amplifikationsansatz. Aufgrund des Perfekt-Match-Controller-Oligonukleotids kann die Matrize amplifiziert werden und es kommt ab Zyklus 27 zu einem Anstieg des Fluoreszenzsignals. Pfeil 2 markiert einen Negativkontrolle-Ansatz, der keine Matrize enthält und daher im Laufe des Experiments auch kein Amplifikationssignal generiert.

Fig. 3B zeigt einen typischen Verlauf des EvaGreen-Fluoreszenzsignals im Laufe einer Allelspezifischen Amplifikationsreaktion unter Verwendung eines Mismatch-Controller-Oligonukleotids. Auf der Y-Achse ist das Fluoreszenzsignal des EvaGreen-Farbstoffes und auf der X-Achse ist die Reaktionszeit (als Zyklusnummer) aufgetragen. Pfeil 1 markiert einen Mismatch-Amplifikationsansatz. Aufgrund des Mismatch-Controller-Oligonukleotids kann die Matrize nicht amplifiziert werden. Pfeil 2 markiert einen Negativkontrolle-Ansatz, der keine Matrize enthält und daher im Laufe des Experiments auch kein Amplifikationssignal generiert. Das Fluoreszenzsignal von Mismatch-Controller-Oligonukleotid-Ansatz und Negativkontrolle sind nahezu deckungsgleich, was zeigt, wie stark die Amplifikation in der Mismatch-Situation unterdrückt wird.

Fig. 3C zeigt die Schmelzkurven der allelspezifischen Amplifikationsprodukte aus Fig. 3A und 3B. Auf der Y-Achse ist die Ableitung des Fluoreszenzsignals gegen die Temperatur und auf der X-Achse ist die Temperatur aufgetragen. Es wurden zwei charakteristische Peaks gefunden, markiert mit Pfeil 1 und 2. Der Peak bei Position 1 mit einem Schmelzpunkt in der Nähe von 65 °C gehört zum Mismatch-Controller-Oligonukleotid-Ansatz. Der Signalverlauf ist nicht von Negativkontroll-Ansätzen (= Amplifikationsreaktion ohne Matrize) zu unterscheiden. Dem gegenüber zu stellen ist der Peak bei Position 2 mit einem Schmelzpunkt in der Nähe von etwa 85 °C. Dieser Peak gehört zum Perfekt-Match-Controller-Oligonukleotid-Ansatz und ist spezifisch für das gebildete Amplifikationsprodukt. Die Ergebnisse legen nahe, dass in der Perfekt-Match-Situation spezifische Amplifikationsprodukte mit einem definierten Schmelzpunkt in der Nähe von etwa 85 °C entstehen. Dagegen wird bei Mismatch-Controller-Oligonukleotiden die Amplifiktionsreaktion unterdrückt. Die Fluoreszenzsignale der Mismatch-Controller-Oligonukleotid-Ansätze waren in diesem Beispiel nicht von der Negativ-Kontrolle zu unterscheiden. Dies zeigt wie stark die Amplifikationsunterdrückung in der Mismatch-Situation ist.

Zusammenfassend wird festgehalten: ein Nukleotid-Mismatch im Controller-Oligonukleotid positioniert im zweiten Bereich des Controller-Oligonukleotides zum korrespondierenden ersten Bereich des Primers kann bei eine Amplifikation verhindern.

Ausgehend von diesem Beispiel kann man erkennen, dass Sequenz-Varianten-spezifische Amplifikations-Systeme umfassend einen Sequenz-Variante-spezifischen Primer und einem entsprechenden komplementären Sequenz-Variante -spezifischen Controller-Oligonukleotid zusammengestellt werden können.

### Beispiel 3

### Primer Verlängerungs-Reaktion unter Verwendung einer Matrize und Block-Oligonukleotiden mit LNA-Modifikationen zu einer Begrenzung einer Primer-Verlängerungsreaktion.

In diesem Beispiel wird die Verwendung humaner genomischer DNA (hgDNA) als Quelle einer Zielsequenz gezeigt, wobei die Primer-Verlängerungsreaktion unter Verwendung von einem ersten Primer-Oligonukleotid und einem Controller-Oligonukleotid durchgeführt wird. Zur Begrenzung des Synthesefortschrittes wurden Block-Oligonukleotide verwendet, welche komplementär an den Matrizen-Strang in 3'-Richtung vom Primer vor dem Start einer Primer-Verlängerungsreaktion durch Hybridisierung gebunden wurden. Die Synthese wurde für 15 min bei 55°C durchgeführt. Während dieser Synthese-Phase bindet der erste Primer-Bereich an die Primer-Bindungsstelle in der Matrize und wird von der Polymerase verlängert, so dass dabei ein Primer-Verlängerungsprodukt resultiert. Der Controller wurde vor Beginn der Primer-Verlängerungsreaktion zugegeben und lag im doppelsträngigen Komplex mit dem Primer während dieser Synthese-Phase in einer nicht-aktiven Form im Ansatz vor.

Nach Abschluss der Synthese-Phase wurde die Reaktionstemperatur auf 65°C für 5 min erhöht, so dass der Komplex umfassend den Controller und den Primer bei dieser Temperatur dissoziieren konnte und der Controller nun mit dem während der Synthese-Phase gebildeten Primer-Verlängerungsprodukt interagieren konnte und es vom Matrizen Strang trennen konnte.

Als Zielsequenz (SEQ ID NO 8) wurde ein Sequenzsegment des Faktor-V-Leiden Genes (Homo sapiens coagulation factor V (F5), mRNA, hier als FVL-Gen bezeichnet) gewählt.

Folgende Komponenten wurde verwendet:
- Block-Oligonukleotid P1-ExB-1000-101 (SEQ ID NO 9);
- erster Primer-Oligonukleotid P1F5-200-AE2053 (SEQ ID NO:3);
- Controller-Oligonukleotid AD-F5-1001-503 (SEQ ID NO: 4).

Der erste Primer sowie der Controller-Oligonukletid wurden für die FVL-Mutation Variante des Genes designet und synthetisiert und umfasst in seinem ersten Bereich eine Sequenz, welche spezifisch an die Sequenz des Faktor-V-Leiden Genes innerhalb der genomischen DNA binden kann, so dass eine Synthese durch eine Polymerase gestartet werden kann. Der zweite Bereich des ersten Primers umfasst eine Sequenz, welche nicht an die Sequenz des FVL-Genes spezifisch hybridisiert. Weiterhin umfasst der erste Primer ein weiteres Sequenzsegment, welches an das 5'-Ende des zweiten Bereichs anknüpft. Dieses Segment nimmt an der spezifischen Primer-Verlängerung des Faktor-5-Leiden Segments nicht teil. Die Funktion dieses Segments wird vor allem in der Verzögerung von Nebenreaktionen gesehen. Das Controller-Oligonukleotid kann mit seinem Segment an das erste zu erwartende Primer-Verlängerungsprodukt (SEQ ID NO 10) komplementär binden. Das Controller-Oligonukleotid wurde derart konstruiert, dass eine Perfekt-Match-Situation zur Sequenz der Faktor-V-Leiden Mutation des FVL-Gens resultiert. Das Controller-Oligonukleotid umfasst einen ersten, zweiten und dritten Bereich. Alle Reaktionen wurden in Amplifikations-Lösung 2 durchgeführt.

Etwa 50000 haploider genomischer Äquivalente (HGE), 150 ng hgDNA in 40 µl, FVL-WHO-Standard, doppelsträngig, wurden in Kontakt mit einem Block-Oligonukleotid (0,3 µmol/l) gebracht und durch Erhitzung zunächst denaturiert (5 min bei 95°C) und anschließend auf Raumtemperatur abgekühlt. Anschließend wurden in die Reaktion der erste Primer (2 µmol/l) zusammen mit Controller-Oligonukleotid (1 µmol/L) zugegeben und anschließend Bst-2.0 Warm Start Polymerase (etwa 1 unit), sowie dNTPs (ca. 250 µmol/l) gegeben und das Reaktionsvolumen auf 50 µl gebracht. Das resultierende Reaktionsgemisch wurde unter stringenten Primer-Verlängerungsbedingungen (Temperatur von etwa 55°C) für ca. 15 min inkubiert. Während dieser Phase erfolgt eine Verlängerung des ersten Primers, wobei die genomische DNA als Matrize diente. Es resultierte ein Primer-Verlängerungsprodukt. Da während dieser Phase das Controller-Oligonukleotid vom ersten Primer komplexiert wird (und somit in einem inaktiven Zustand vorliegt), lag das gebildete Primer-Verlängerungsprodukt im doppelsträngigen Komplex mit der Matrize vor. Um dieses Produkt von der Matrize sequenzspezifisch abzulösen, wurde das Reaktionsgemisch auf 65°C erhitzt und ca. 5 min inkubiert. Während dieser Phase wurde das Primer-Verlängerungsprodukt aus der Bindung mit der Matrize durch den Controller zumindest teilweise verdrängt. Das 3'-Segment des Primer-Verlängerungsproduktes dissoziierte spontan unter gewählten Reaktionsbedinungen. Ein solches Primer-Verlängerungsprodukt kann beispielsweise in einer nachfolgenden Amplifikations-Reaktion in Analogie zum Beispiel 1 verwendet werden.

Eine Anordnung der Block-Oligonukleotide an einer Matrize, welche eine Begrenzung der Länge eines Primer-Verlängerungsproduktes verursacht, kann beispielsweise bei Bereitstellung eines Nukleinsäure-Fragmentes verwendet werden, welches als Start-Nukleinsäure in einer spezifischen exponentiellen Amplifikation verwendet werden soll. Zwar kann eine solche Start-Nukleinsäure als Primer-Verlängerungs-Reaktion auch ohne ein Block-Oligonukleotid erfolgen. Die undefinierte Länge eines Primer-Verlängerungsfragments, welches beim Kopieren des Matrizenstranges dabei entstehen kann, kann allerdings eine sequenz-unspezifische, z.B. eine temperatur-bedingte Strang-Trennung erforderlich machen. Die Bindung eines zweiten Amplifikations-Primers erfolgt dabei im mittleren Bereich eines solchen Primer-Verlängerungsproduktes.

Die Verwendung eines Block-Oligonukleotides erlaubt dagegen eine beinahe willkürliche spezifische Begrenzung der Länge eines Primer-Verlängerungsproduktes, ungeachtet der Länge eines Matrizenstranges. Weiterhin begünstigt das Block-Oligonukleotid eine sequenzabhängige Trennung von gebildeten Primer-Verlängerungsprodukten bereits vor Beginn einer Amplifikation. Dabei erfolgt beispielsweise zunächst eine Synthese-Phase einer Primer-Verlängerung und anschließend eine Controlling-Phase (Sequenzielle Anordnung von Schritten). Auch kann die Synthese-Phase und die Controlling-Phase mit der Trennung des Primer-Verlängerungsproduktes in einem Schritt ablaufen und parallel zu einander stattfinden. Im Ergebnis wird ein spezifisches Primer-Verlängerungsprodukt erzeugt und sequenzspezifisch von der Matrize unter Mitwirkung eines Controllers getrennt, ohne dabei einen unspezifischen Denaturierungsschritt einsetzten zu müssen. Ein solches Primer-Verlängerungsprodukt kann als Start-Nukleinsäure für eine weitere Amplifikation verwendet werden. Dadurch kann die Spezifität der Erstellung einer Start-Nukleinsäure erhöht werden.

Das Block-Oligonukleotid dient vorzugsweise selbst nicht als Primer. Das kann beispielsweise durch Blockade seiner 3'-OH Gruppe erfolgen. Das Block-Oligonukleotid umfasst somit mindestens ein Sequenzsegment, welches zum Matrizen-Strang komplementär ist, an welchem auch die Primer-Verlängerungs-Reaktion erfolgt. Dieses Segment (Blockierendes Segment) ist vorzugsweise hinreichend lang bzw. umfasst ggf. Modifikationen, um bei Reaktionsbedingungen einer Primer-Verlängerungsreaktion mit dem Matrizenstrang einen stabilen Doppelstrang zu bilden. Weiterhin kann ein solches Block-Oligonukloetid auch Sequenzsegmente umfassen, welche nicht zum Matrizen-Strang komplementär sind. Solche Sequenzsegmente können beispielsweise das Segment, welches an die Matrize binden soll und die Primer-Verlängerungs-Reaktion blockieren soll, auf beiden Seiten flankieren. Das Block-Oligonukleotid kann beispielsweise in einem Sequenzsegment einer Zielnukleinsäure lokalisiert sein oder an einem ihrer Enden platziert werden. Das Block-Oligonukleotid ist somit derart angeordnet, dass es in 3'-Richtung vom zu erwartenden Primer-Verlängerungsprodukt an der Matrize lokalisiert ist und die Polymerase am weiteren Kopieren des Matrizenstranges hindert.

Das Ziel einer Primer-Verlängerungsreaktion kann die Herstellung einer Start-Nukleinsäurekette für eine anschließende Amplifikations-Reaktion darstellen. Eine solche Start-Nukleinsäurekette wird zu Beginn einer Amplifikations-Reaktion eingesetzt. Ihre Funktion kann dahingehend gesehen werden, dass sie die anfängliche Matrize darstellt, welche eine korrekte Positionierung von Primern, der Synthese-Abschnitte zwischen beiden Primern, sowie die Initiierung von Bindungs- und Verlängerungsvorgängen ermöglicht. In einer bevorzugten Ausführungsform umfasst eine Start-Nukleinsäurekette eine Zielsequenz.

Durch Bindung von Primern an ihre entsprechenden Primer-Bindungsstellen (PBS 1 und PBS 2) und Initiierung von entsprechenden Primer-Verlängerungsreaktionen erfolgt durch Generierung von ersten Primer-Verlängerungsprodukten. Diese werden als spezifische Kopien der zu Beginn der Reaktion vorliegenden Nukleinsäurekette synthetisiert.

### Beispiel 4

### Einfluss eines Controller-Oligonukleotids auf die Primer-Verlängerungs-Reaktion bei einer parallel stattfindenden Synthese-Phase und Controlling-Phase:

Folgende Matrizen wurden verwendet (DNA-Matrizen):
- P1Ex-400-4001 (SEQ ID NO 11)
- P1EX-400-4002 (SEQ ID NO 12)
- P1EX-400-4003 (SEQ ID NO 13)
- P1EX-400-4004 (SEQ ID NO 14)
- P1EX-400-4005 (SEQ ID NO 15)
- P1EX-400-4201 (SEQ ID NO 16)
- P1EX-400-4202 (SEQ ID NO 17)

Die Matrizen wurden dermaßen in ihrer Sequenzzusammensetzung variiert, so dass daraus resultierende Primer-Verlängerungsprodukte unterschiedlich mit dem Controller-Oligonukleotid interagieren können. Je nach Sequenzzusammensetzung der Matrize konnte somit entweder eine perfekt-match Übereinstimmung oder auch ein Mismatch im Komplex aus Primer-Verlängerungsprodukt / Controller generiert werden.

Zur Überprüfung eines Polymerase-Fehlers wurden Matrizen verwendet, welche an bestimmten Positionen ein Iso-dC (Nukleotid kann nur mit Iso-dG eine komplementäre Nukleobase bilden. Es bildet keine "typische Watson-Crick" Basenpaarung mit einem der verwendeten dNTP's, so dass die Überwindung dieser Position nur Mismatch-Bildung erfolgen kann) einschließen. Da dieses Nukleotid kein natürliches Korrelat umfasst, muss eine Polymerase ein Mismatch generieren und erst bei Überwindung dieses Mismatches kann die Verdrängung der Sonde (s.u.) stattfinden. Somit repräsentieren Matrizzen mit Iso-dC ein Beispiel für die Primer-Verlängerungs-Reaktionen, welche unter einer Mismatch-Bildung (z.B. infolge eines Polymrease-Fehlers) entstehen. Matrizen mit Iso-dC sollen lediglich die Änderungen im Verhalten der Primer-Verlängerungs-Reaktionen veranschaulichen und den Einfluss vom Controller auf das Verhalten der Polymerasen bzw. der Synthese von komplementären Strängen.

Folgende Komponentent wurden verwendet:
- SeqP1F5 300-35x (SEQ ID NO 18), Primer umfasst kein Segment, welches mit dem ersten Bereich des Controller-Oligonukleotides interagieren kann. Somit kann ein Controller-Oligonukleotid bei Verlängerung dieses Primers das gebildete Primer-Verlängerungsprodukt nicht vom Matrizenstrang trennen;
- Primer P1F5-001-1003 (SEQ ID NO 19);
- Controller-Oligonukleotid AD-F5-1001-503 (SEQ ID NO 20), Controller-Oligonukleotid kann in diesem Beispiel mit bestimmten Primer-Verlängerungsprodukten ein perfekt-match bilden. Mit anderen Primer-Verlängerungsprodukten kann es ein Mismatch bilden;
- Fluoreszenz-Sonde P2D-LUX-1000-101 (SEQ ID NO 21), bindet an das 5'-Segment der verwendeten Matrizen, bei Verdrängung dieser Sonde aus der Bindung mit diesem 5'-Segment der jeweiligen Matrize kommt es zu Minderung des Signals der Sonde (Intramolekulares Quenchinng zwischen dT-BHQ1 und FAM-Reporter).

Die Sonde wurde in 0,2 µmol/L Konzentration verwendet. Die Matrizen wurden in Konzentration von 0,5 µmol/l verwendet, die Primer wurden in 1 µmol/l Konzentration eingesetzt und Controller (wenn verwendet, wie indiziert) wurden bei 2 µmol/l verwendet. Bst-2.0 Warm-Start (NEB) wurde in Konzentration eingesetzt 4 Units / 10 µl.

Die Reaktionen wurden in StepOne Plus Gerät durchgeführt bei Temperatur von 55°C. Die Signal-Detektion erfolgte via FAM-Kanal. Die Reaktion wurde kontinuierlich überwacht (Sampling-Interval = 10 sec). Als Kontrolle der Signal-Intensität dienten zum einen Reaktions-Ansätze ohne Polymerase (aber mit allen anderen Komponenten) oder alternativ mit Polymerase aber ohne Matrize.

Die Tm von PBS (perfekt match) der Matrize mit dem ersten Bereich des Primers betrug ca. 45°C. Die Tm von Controller-Oligonukleotid / Primer (P1F5-001-1003) betrug ca. 57°C. Die Reaktionstemperatur der Primer-Verlängerungs-Reaktion war 55°C. Die Reaktion verlief über 100 min.

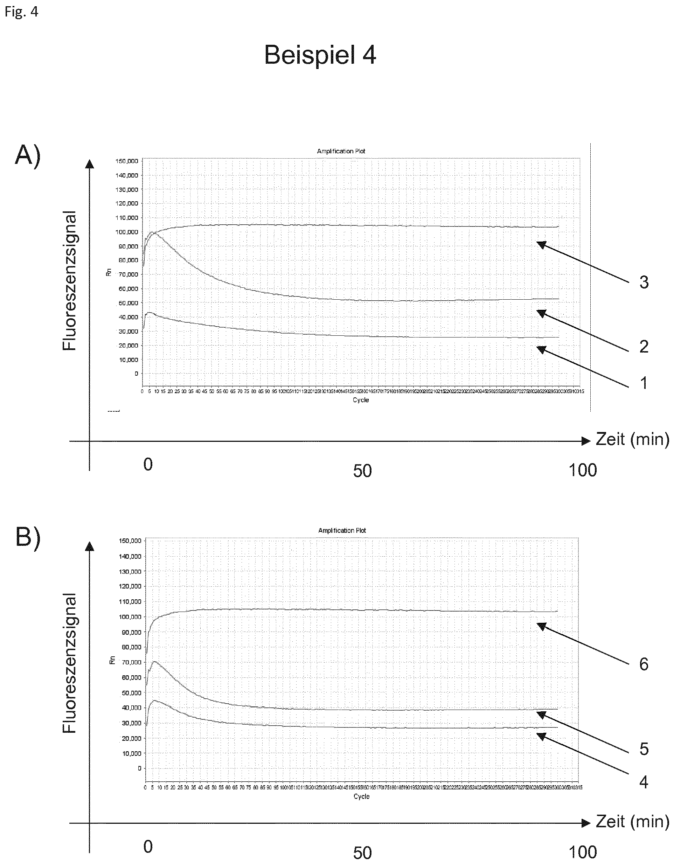

Die Ergebnisse von einzelnen Reaktionen sind in Fig. 4 zusammengefasst.

Die Kinetik einzelner Reaktionen wurde anhand der Veränderung von Fluoreszenzsignal ausgewertet.

Nachfolgend wird die Auswertung von representativen Datensätzen dargestellt:

| Reaktion | Matrize | Primer | Controller | Polymerase |
|---|---|---|---|---|
| 1 | P1Ex-400-4001 | P1F5-001-1003 | nein | Ja |
| 2 | P1Ex-400-4001 | P1F5-001-1003 | AD-F5-1001-503 | ja |
| 3 | P1Ex-400-4001 | P1F5-001-1003 | nein | nein |

Reaktion 1 (Fig. 4 A): Bei perfekt-match Bindung eines Primers in Abwesenheit eines Controllers erfolgte eine rasche Primer-Verlängerung und Sonden-Verdrängung, so dass bereits bei der ersten Signal-Erfassung mehr als 70% der Sonden vom 5'-Segment der Matrizen abgelöst waren und das Signal stark gemindert war bereits zu Beginn der Messung.
Reaktion 2 (Fig. 4 A): Bei perfekt-match Bindung eines Primers in Anwesenheit eines Controllers erfolgte ebenfalls eine schnelle Primer-Verlängerung und Sonden-Verdrängung, welche allerdings langsamer verläuft verglichen mit Reaktion 1 ohne Controller.
Reaktion 3 (Fig. 4 A): diente als Referenz-Signal für Sonde in Abwesenheit einer Polymerase.

| Reaktion | Matrize | Primer | Controller | Polymerase |
|---|---|---|---|---|
| 4 | P1Ex-400-4001 | SEQP1F5 300-35X | nein | Ja |
| 5 | P1Ex-400-4001 | SEQP1F5 300-35X | AD-F5-1001-503 | ja |
| 6 | P1Ex-400-4001 | SEQP1F5 300-35X | nein | nein |

Reaktionen 4 - 6 (Fig. 4 B) zeigen Reaktionen mit einem Primer, welches nicht mit dem Controller im ersten Bereich interagieren kann (dieser Primer hat keinen zweiten Bereich, welches komplementär an den ersten Bereich des Controllers binden kann). Die Anwesenheit des Controllers scheint keinen detektierbaren Einfluss auf den Verlauf dieser Reaktion zu haben. Bei gewählten Reaktionsbedinungen stört der Controller den Verlauf dieser Reaktion kaum.
Einführung eines Mismatches in Primer-Bindungs-Stelle in der Matrize

| Reaktion | Matrize | Primer | Controller | Polymerase |
|---|---|---|---|---|
| 7 | P1EX-400-4002 | P1F5-001-1003 | nein | Ja |
| 8 | P1EX-400-4002 | P1F5-001-1003 | AD-F5-1001-503 | ja |
| 9 | P1EX-400-4002 | P1F5-001-1003 | nein | nein |

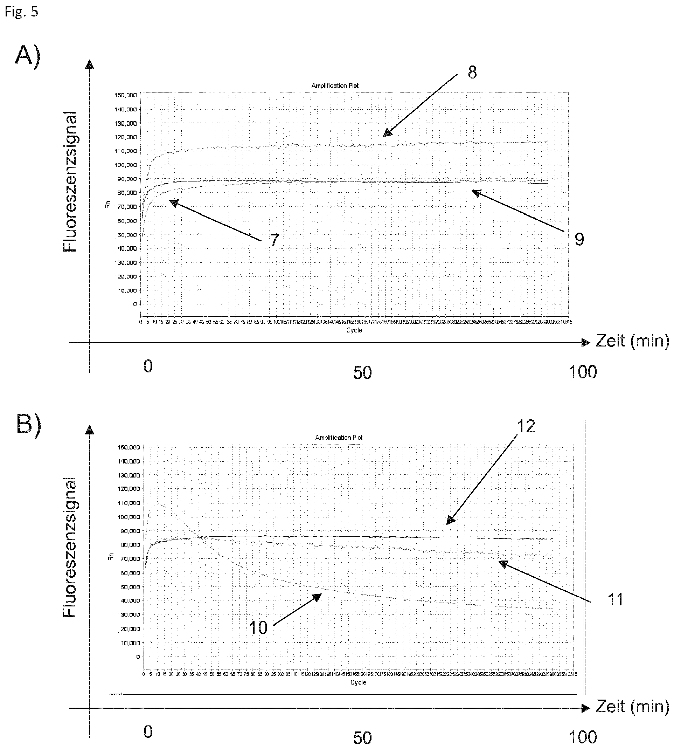

Reaktionen 7 und 8 zeigen, dass das Fehlen eines komplementären Nukleotides in der Matrize (3'-terminales Mismatch) zu einer vollständigen Verhinderung der Primer-Verlängerungs-Reaktion führt (Fig. 5A). Analoges Reaktionsverhalten wurde bei Matrizen P1EX-400-4003, P1EX-400-4004, P1EX-400-4005, P1EX-400-4201 beobachet (Fehlen von 2 terminalen Nukleotiden, ein Mismatch in der Mitte von PBS, eine Substitution von G auf A (Mismatch), eine Substitution von G auf Iso-dC). In keiner der Reaktionen wurde die Sonde verdrängt. Das zeigt, dass sich die Anwesenheit von Controller-Oligonukleotid nicht negativ auf die Diskriminierung auswirkt.

| Reaktion | Matrize | Primer | Controller | Polymerase |
|---|---|---|---|---|
| 10 | P1EX-400-4202 | P1F5-001-1003 | nein | Ja |
| 11 | P1EX-400-4202 | P1F5-001-1003 | AD-F5-1001-503 | ja |
| 12 | P1EX-400-4202 | P1F5-001-1003 | nein | nein |

Reaktion 10 (Fig. 5 B): Zeigt Überwindung einer Iso-dC-Position in der Matrize in Abwesenheit eines Controllers und Fortsetzunng der Synthese bis zur Verdrängung der Sonde unter Verwendung eines ersten Primer-Oligonukleotides (P1F5-001-1003). Man sieht zwar eine verzögerte Reaktionskinetik (Verdrängung der Sonde erfolgt wesentlich langsamer, als bei perfekt-Match Matrize), dennoch kann ein komplementäres Primer-Verlängerungsprodukt in guten Ausbeuten synthetisiert weren (Sonde wurde verdrängt).
Reaktion 11 (Fig. 5 B): Zeigt Einfluss des in der Reaktion anwesenden Controllers: Die Überwindung der Mismatch-Position gelingt wesentlich langsamer. Dies wird damit in Verbindung gebracht, dass das während der Reaktion entstehende Primer-Verlängerungsprodukt während der Reaktion abgelöst werden kann und mit dem Controller einen Komplex bildet, welcher eine höhere Tm hat, als Primer / Controller-Complex. Das vorzeitig abgelöste, nicht vollständige Primer-Verlängerungsprodukt verbleibt bevorzugt im Komplex mit Controller und wird damit aus der Reaktion entzogen ("Trapping" von unvollständigen Primer-Verlängerungsprodukten). Damit wird verhindert, dass die Polymerase das Iso-dC Nukleotid überwinden kann. Die Anwesenheit des Controllers übt somit eine "korrigierende Wirkung" auf die Polymerase aus: zwar ist eine Polymerase grundsätzlich in der Lage, ein solches Mismatch zu überwinden, doch in Kombination mit Controller wird diese Eigeschaft des Primer-Verlängerungs-Systems dermaßen verändert, dass eine vollständige Synthese von fehlerhaften Primer-Verlängerungsprodukten unterdrückt wird. Die zwischenzeitlich entstehenden Primer-Verlängerungsprodukte werden aus der weiteren Verlängerung entzogen während eine Primer-Verlängerungs-Reaktion läuft (Online Kontrolle).
Reaktion 12: diente als Referenz-Signal für Sonde in Abwesenheit einer Polymerase.

| Reaktion | Matrize | Primer | Controller | Polymerase |
|---|---|---|---|---|
| 13 | P1EX-400-4202 | SEQP1F5 300-35X | nein | Ja |
| 14 | P1EX-400-4202 | SEQP1F5 300-35X | AD-F5-1001-503 | ja |
| 15 | P1EX-400-4202 | SEQP1F5 300-35X | nein | nein |

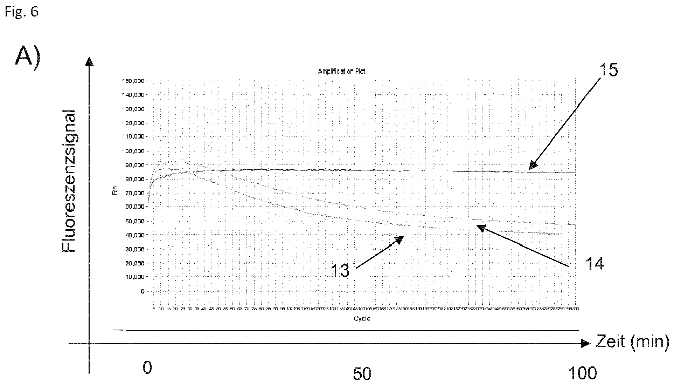

Reaktion 13 (Fig. 6 A): Zeigt Überwindung einer Iso-dC-Position in der Matrize in Abwesenheit eines Controllers und Fortsetzunng der Synthese bis zur Verdrängung der Sonde unter Verwendung eines Primer-Oligonukleotides, welches keinen zweiten Bereich umfasst (SEQP1F 300-35X). Man sieht zwar eine verzögerte Reaktionskinetik (Verdrängung der Sonde erfolgt wesentlich langsamer, als bei perfekt-Match Matrize), dennoch kann ein komplementäres Primer-Verlängerungsprodukt in guten Ausbeuten synthetisiert werden (Sonde wurde verdrängt).
Reaktion 14: Die Anwesenheit eines Controller-Oligonukleotides scheint bei einem solchen Primer-Design allerdings keine Auswirkung auf das Verhalten des Primer-Verlängerungs-Systems zu haben: der verwendete Primer hat keinen zweiten Bereich, im Gegensatz zum in der Reaktion 11 verwendeten Primer.

Insgesamt wurde in diesem Beispiel die Fähigkeit eines Primer-Verlängerungs-Systems demonstriert, die Überwindung eines Mismatch durch die Polymerase in Anwesenheit eines Controller-Oligonukleotides und eines ersten Primers zu modifizieren / zu verhindern.

## Patentansprüche

1. Ein Verfahren zur Synthese einer zu einer Zielsequenz komplementären Nukleinsäuresequenz, wobei
a) eine Probe, die ein Nukleinsäurepolymer umfassend die Zielsequenz M umfasst, wobei die Zielsequenz in 5'-3'-Orientierung die Sequenzabschnitte MS und MP umfasst und MP unmittelbar in 3' von MS angeordnet ist, mit folgenden Komponenten in Kontakt gebracht wird:
b) ein Oligonukleotidprimer P, welcher in 5'-3'-Orientierung die Sequenzabschnitte PC und PM umfasst und PM unmittelbar in 3' von PC angeordnet ist, wobei PM die zum Sequenzabschnitt MP komplementäre [die hybridisierende] Sequenz aufweist [im Wesentlichen sequenzspezifisch binden kann] und PC nicht an M [oder eine in 3' unmittelbar an MP anschließende Sequenz] binden kann;
c) ein Controller-Oligonukleotid C, welches in 5'-3'-Orientierung die Sequenzabschnitte CS, CP und CC umfasst, wobei CP unmittelbar in 3' von CS und unmittelbar in 5' von CC angeordnet ist, und wobei eine Nukleinsäuresequenz am 3'Endevon CS mit einer Länge von mindestens 10 Nukleotiden, insbesondere die gesamte Nukleinsäuresequenz von CS, identisch zu einer Nukleinsäuresequenz am 3'Ende von MS identisch ist und die Nukleinsäuresequenz von CP identisch zur Nukleinsäuresequenz von MP ist und CC die zum Sequenzabschnitt PC komplementäre [die hybridisierende] Sequenz aufweist und wobei CS und / oder CP modifizierte Nukleotidbausteine umfasst, so dass CS nicht als Matrize für die Aktivität der matrizenabhängigen Nukleinsäurepolymerase dienen kann;
d) eine matrizenabhängige Nukleinsäurepolymerase, insbesondere eine DNA-Polymerase, sowie Substrate der matrizenabhängigen Nukleinsäurepolymerase (insbesondere Ribonukleosid-triphosphate oder Desoxyribonukleosid-triphosphate) und geeignete Kofaktoren,
wobei ein Primer-Verlängerungsprodukt P' als Ergebnis einer matrizenabhängigen Verlängerung des Primers P durch die matrizenabhängige Nukleinsäurepolymerase erhalten wird, welches neben den Sequenzbereichen PC und PM einen synthetisierten Bereich PS umfasst, der im Wesentlichen komplementär zur Zielsequenz MS ist,
und wobei
- entweder die Reaktionsbedingungen so gewählt sind, und/oder
- die Länge und Schmelztemperatur von PC und/oder MS so gewählt sind,
dass P' mit M einen Doppelstrang bilden kann und mindestens ein Teil von P' mit C einen Doppelstrang bilden kann und die Bildung des Doppelstranges aus P' und C gegenüber der Bildung des Doppelstrangs aus P' mit M bevorzugt ist.

2. Das Verfahren gemäß Anspruch 1, wobei der Sequenzabschnitt CS an seinem 3' Ende unmittelbar 5' des Sequenzabschnitts CP einen Sequenzabschnitt CS' von 5 bis 50 Nukleotidpositionen Länge enthält, welcher aus Nukleinsäureanaloga, insbesondere aus 2'O-Alkylribonukleotiden, besteht.

3. Das Verfahren gemäß einem der vorstehenden Ansprüche, wobei M einen Sequenzabschnitt MB unmittelbar in 5' von MS umfasst, und wobei weiterhin ein Block-Oligonukleotid B, das an MB hybridisieren kann, mit der Probe in Kontakt gebracht wird, und wobei
a. B Nukleosidanaloga umfasst, die so ausgewählt sind, dass ein Hybrid von B mit MB die Reaktion der matrizenabhängigen Nukleinsäurepolymerase auf MB nicht erlaubt, oder
b. die matrizenabhängige Nukleinsäurepolymerase so ausgewählt ist, dass ein Hybrid von B mit MB [auch wenn B aus natürlichen Nukleosiden oder Deoxynukleosiden besteht] die Reaktion der matrizenabhängigen Nukleinsäurepolymerase auf MB nicht erlaubt; und
wobei insbesondere B mit der Probe in Kontakt gebracht wird, bevor die matrizenabhängige Nukleinsäurepolymerase mit der Probe in Kontakt gebracht wird.

4. Das Verfahren nach einem der vorherigen Ansprüche, **dadurch gekennzeichnet, dass** das Verfahren im Wesentlichen isothermal durchgeführt wird; und wobei insbesondere die gewählten Reaktionsbedingungen eine Reaktionstemperatur im Bereich von 25 °C bis 80 °C umfassen.

5. Das Verfahren gemäß einem der vorstehenden Ansprüche, wobei die Probe **nicht** mit einem Primer-Oligonukleotid in Kontakt gebracht wird (oder dieses umfasst), welches an einen in PS gelegenen Sequenzabschnitt binden und von dem aus durch die matrizenabhängige Nukleinsäurepolymerase eine Synthese eines Gegenstrangs zu PS erfolgen kann (mit anderen Worten: das Verfahren führt nicht zur exponentiellen Amplifikation von M).

6. Das Verfahren nach einem der vorherigen Ansprüche, **dadurch gekennzeichnet, dass**
- MS eine Länge von 20 Nukleotiden bis 200 Nukleotiden hat; und/oder
- P eine Länge im Bereich von 15 Nukleotiden bis 100 Nukleotiden hat; und/oder
- PC eine Länge im Bereich von 5 Nukleotiden bis 85 Nukleotiden hat, insbesondere dass PC zwischen 50% und 300% der Länge des Sequenzabschnitts PM hat; und/oder
- C eine Länge im Bereich von 20 Nukleotiden bis 100 Nukleotiden hat.

7. Das Verfahren nach einem der vorherigen Ansprüche, durch gekennzeichnet, dass die Probe eine Variante VM der Zielsequenz M umfasst, welche ≥85%, insbesondere ≥95%, ≥92%, ≥94%, ≥96% oder sogar ≥98% identisch zu M ist, wobei
- ein Abschnitt VMS der Variante dem Abschnitt MS hoch identitisch ist, wobei insbesondere die Abschnitte MS und VMS ≥90%, insbesondere ≥98%, ≥99% oder sogar 100% identisch sind, und
- ein Abschnitt VMP dem Abschnitt MP hochidentisch ist, wobei insbesondere die Abschnitte MP und VMP ≥90%, insbesondere ≥98%, ≥99% oder sogar 100% identisch sind, und
- VM in mindestens einer Position VMM von der Sequenz M verschieden ist, insbesondere eine oder mehrere Substitution(en), Insertion(en) und/oder Deletion(en) aufweist;
wobei insbesondere die mindestens eine Position VMM eine Länge von 1 bis 20 Nukleotiden, insbesondere von 1 bis 3 Nukleotiden, aufweist; und wobei insbesondere VMM in dem von VMS umfassten Sequenzabschnitt liegt, und wobei insbesondere die Abschnitte MS und VMS ausserhalb des Abschnitts VMM ≥90%, ≥95% oder insbesondere 100%, identisch sind.

8. Das Verfahren nach Anspruch 7, **dadurch gekennzeichnet, dass** VMM < 50 Nukleotidpositionen, insbesondere <25, <15 oder <10 Nukleotidpositionen in 5' von VMP auf VM angeordnet ist, und wobei insbesondere VMM mindestens teilweise von dem von VMP umfassten Sequenzabschnitt umfasst wird.

9. Das Verfahren nach einem der vorherigen Ansprüche 7 bis 8, **dadurch gekennzeichnet, dass** weiterhin ein zweites Controller-Oligonukleotid VC mit der Probe in Kontakt gebracht wird, welches in 5'-3'-Orientierung die Sequenzabschnitte VCS, VCP und VCC umfasst, wobei VCP unmittelbar in 3' von VCS und unmittelbar in 5' von VCC angeordnet ist, und wobei eine Nukleinsäuresequenz am 3'Endevon VCS mit einer Länge von mindestens 10 Nukleotiden, insbesondere die gesamte Nukleinsäuresequenz von VCS, mit einer Nukleinsäuresequenz am 3'Ende von VMS identisch ist und die Nukleinsäuresequenz von VCP identisch mit MP ist und VCC die zum Sequenzabschnitt PC komplementäre [die hybridisierende] Sequenz aufweist und wobei VCS und / oder VCP modifizierte Nukleotidbausteine umfasst, so dass VCS nicht als Matrize für die Aktivität der matrizenabhängigen Nukleinsäurepolymerase dienen kann.

10. Das Verfahren nach einem der vorherigen Ansprüche 7 bis 9, **dadurch gekennzeichnet, dass** weiterhin ein zweiter Oligonukleotidprimer VP mit der Probe in Kontakt gebracht wird, welcher in 5'-3'-Orientierung die Sequenzabschnitte VPC und VPM umfasst und VPM unmittelbar in 3' von VPC angeordnet ist, wobei VPM die zum Sequenzabschnitt VMP komplementäre [die hybridisierende] Sequenz aufweist [im Wesentlichen sequenzspezifisch binden kann] und VPC nicht an VM [oder eine in 3' unmittelbar an VMP anschließende Sequenz] binden kann.

11. Das Verfahren nach einem der vorherigen Ansprüche 7 bis 10, **dadurch gekennzeichnet, dass** VMM am 5'-Ende von VMP liegt, mit anderen Worten, dass das 3'-Ende von VP mit VMM hybridisiert.

12. Das Verfahren nach einem der vorherigen Ansprüche 7 bis 11, **dadurch gekennzeichnet, dass** weiterhin ein Kompetitor-Oligonukleotid KP mit der Probe in Kontakt gebracht wird, welches mit VMP hybridisieren kann.

13. Ein Kit zur Durchführung eines Verfahrens nach einem der Ansprüche 1 bis 12, umfassend:
- ein Oligonukleotidprimer P, welcher in 5'-3'-Orientierung die Sequenzabschnitte PC und PM umfasst und PM unmittelbar in 3' von PC angeordnet ist, wobei PM an eine Sequenz M eines eukaryoten Organismus oder eines pathogenen Bakteriums, insbesondere eines Säugetiers, ganz inbesondere an eine genomische menschliche Zielsequenz, umfassend eine Primerbindungsstelle MP binden kann und PC nicht an eine direkt in 3' von MP liegende Sequenz binden kann;
- ein Controller-Oligonukleotid C, welches in 5'-3'-Orientierung die Sequenzabschnitte CS, CP und CC umfasst, wobei CP unmittelbar in 3' von CS und unmittelbar in 5' von CC angeordnet ist, und wobei eine Nukleinsäuresequenz von CS identisch mit einer unmittelbar in 5' von MP liegenden Sequenz MS über eine Länge von mindestens 10 Nukleotiden, insbesondere über die gesamte Nukleinsäuresequenz von CS ist und die Nukleinsäuresequenz von CP identisch zur Nukleinsäuresequenz von MP ist und CC die zum Sequenzabschnitt PC komplementäre [die hybridisierende] Sequenz aufweist, und wobei C (in seinem CS und / oder CP) modifizierte Nukleotidbausteine umfasst, so dass CS nicht als Matrize für die Aktivität einer matrizenabhängigen Nukleinsäurepolymerase dienen kann.

14. Das Kit gemäß Anspruch 13, weiterhin umfassend ein Block-Oligonukleotid B, das an einen unmittelbar 5' von MS gelegenen Sequenzabschnitt der Zielsequenz MB hybridisieren kann, wobei B Nukleosidanaloga umfasst, die so ausgewählt sind, dass ein Hybrid von B mit MB die Reaktion einer matrizenabhängigen Nukleinsäurepolymerase auf MB nicht erlaubt.

15. Das Kit gemäß Anspruch 13 oder 14, weiterhin umfassend
- einen zweiten Oligonukleotidprimer VP, welcher in 5'-3'-Orientierung die Sequenzabschnitte VPC und VPM umfasst und VPM unmittelbar in 3' von VPC angeordnet ist, wobei VPM an eine genomische Sequenz VM umfassend eine Primerbindungsstelle VMP binden kann, wobei VM zu M ≥85%, insbesondere ≥95%, ≥92%, ≥94%, ≥96% oder sogar ≥98% identisch zu M, aber in mindestens einer Position verschieden von M ist, und VPC nicht an eine direkt in 3' von VMP liegende Sequenz binden kann;
und/oder
- ein zweites Controller-Oligonukleotid VC, welches in 5'-3'-Orientierung die Sequenzabschnitte VCS, VCP und VCC umfasst, wobei VCP unmittelbar in 3' von VCS und unmittelbar in 5' von VCC angeordnet ist, und wobei VCS identisch mit einer (unmittelbar in 5' von VMP liegenden) genomischen Sequenz VMS ist, die Teil einer Variante VM ist, die ≥85%, insbesondere ≥95%, ≥92%, ≥94%, ≥96% oder sogar ≥98% identisch zu M, aber in mindestens einer Position verschieden von M ist, und VCP identisch mit VMP ist und VCC die zum Sequenzabschnitt VPC komplementäre [die hybridisierende] Sequenz aufweist, und wobei VCS und /oder VCP modifizierte Nukleotidbausteine umfasst, so dass VCS nicht als Matrize für die Aktivität einer matrizenabhängigen Nukleinsäurepolymerase dienen kann,
und/oder
- ein Kompetitor-Oligonukleotid KP, welches mit VMP hybridisieren kann, vorzugsweise vorwiegend komplementär t,
- wobei insbesondere dieser Kit **kein** Primer-Oligonukleotid umfasst, welches an einen auf dem Gegenstrang von M [Strang komplentär zu M] gelegenen Sequenzabschnitt binden und von dem aus durch die matrizenabhängige Nukleinsäurepolymerase eine Synthese einer im Wesentlichen zu dem 5' von MP auf M gelegenen Sequenz identischen Sequenz erfolgen kann (mit anderen Worten: der Kit ermöglicht nicht, die 5' von MP auf M gelegenen Sequenz exponentiell zu amplifizieren).


SEQUENCE LISTING

<110>  AGCT GmbH

<120>  Verfahren zur Primer-Verlängerungsreaktion mit verbesserter
       Spezifität

<130>  agc104ep-1

<160>  21

<170>  PatentIn version 3.5

<210>  1
<211>  67
<212>  DNA
<213>  Homo sapiens



<220>
<221>  misc_feature
<222>  (51)..(62)
<223>  Segement 51 bis 62 dient als Primer-Bindungsstelle für den ersten
       Primer

<400>  1
gctcatacta caatgtcact tactgtaaga gcagatccct ggacaggcaa ggaatacagg      60

taaaaaa                                                                67


<210>  2
<211>  67
<212>  DNA
<213>  Homo sapiens



<220>
<221>  misc_feature
<222>  (49)..(49)
<223>  Base mit einem Missmatch zum Controller-Oligonukleotid

<220>
<221>  misc_feature
<222>  (51)..(62)
<223>  Segement 51 bis 62 dient als Primer-Bindungsstelle für den ersten
       Primer

<400>  2
gctcatacta caatgtcact tactgtaaga gcagatccct ggacaggcga ggaatacagg      60

taaaaaa                                                                67


<210>  3
<211>  57
<212>  DNA
<213>  Artificial Sequence

<220>
<223>  Primer für PCR

<220>
<221>  misc_feature
<222>  (34)..(45)
<223>  das Segement 34 bis 45 enthält 2´-O-Me-Modifikationen und dient
      als zweiter Primer-Bereich zur Bindung des ersten Bereich des
      Controller-Oligonukleotides

<220>
<221>  misc_feature
<222>  (44)..(45)
<223>  Zwischen Nukleotid 44 und 45 befindet sich ein C3-Linker, der der
      Terminierung der Polymerase dient

<220>
<221>  misc_feature
<222>  (45)..(57)
<223>  Das Segement 45 bis 57 dient als Primer

<220>
<221>  modified_base
<222>  (45)..(45)
<223>  Uracil instead of thymidine

<220>
<221>  misc_feature
<222>  (45)..(46)
<223>  Zwischen Nukleotid 45 und 46 befindet sich ein C3-Linker, der der
      Terminierung der Polymerase dient

<220>
<221>  modified_base
<222>  (47)..(47)
<223>  Uracil instead of thymidine

<220>
<221>  modified_base
<222>  (50)..(50)
<223>  Uracil instead of thymidine

<220>
<221>  modified_base
<222>  (53)..(53)
<223>  Uracil instead of thymidine

<220>
<221>  modified_base
<222>  (54)..(54)
<223>  Uracil instead of thymidine

<400>  3
aactcagaca agatgtgatt tttttacctg tatctctgat gcttctacct gtattcc          57


<210>  4
<211>  56
<212>  DNA
<213>  Artificial Sequence

<220>
<223>  Controller


<220>

<221>  misc_feature
<222>  (1)..(39)
<223>  Das Segment 1 bis 39 umfasst 2´-O-Me-Nukleotid-Modifikationen

<220>
<221>  modified_base
<222>  (1)..(1)
<223>  Uracil instead of thymidine

<220>
<221>  modified_base
<222>  (4)..(4)
<223>  Uracil instead of thymidine

<220>
<221>  modified_base
<222>  (6)..(6)
<223>  Uracil instead of thymidine

<220>
<221>  modified_base
<222>  (8)..(8)
<223>  Uracil instead of thymidine

<220>
<221>  modified_base
<222>  (18)..(18)
<223>  Uracil instead of thymidine

<220>
<221>  modified_base
<222>  (22)..(22)
<223>  Uracil instead of thymidine

<220>
<221>  modified_base
<222>  (37)..(37)
<223>  Uracil instead of thymidine

<220>
<221>  misc_feature
<222>  (56)..(56)
<223>  Das Ende des Controllers enthält eine 3´-Phosphat-Gruppe zur
      Blockade einer möglichen Verlängerung durch Polymerase

<400>  4
taatctgtaa gagcagatcc ctggacaggc aaggaataca ggtagaagca tcagag          56


<210>  5
<211>  70
<212>  DNA
<213>  Artificial Sequence

<220>
<223>  Primer für PCR


<220>
<221>  misc_feature
<222>  (35)..(36)
<223>  Nukleotid 35 und 36 sind verbunden durch einen HEG-Linker

<220>
<221>  misc_feature
<222>  (36)..(70)
<223>  Segment 36 bis 70 dient als Primer in der Reaktion

<400>  5
ctacagaact cagacaagat gtgaactaca atgttgctca tactacaatg tcacttactg      60

taagagcaga                                                              70


<210>  6
<211>  70
<212>  DNA
<213>  Artificial Sequence

<220>
<223>  Primer für PCR



<220>
<221>  misc_feature
<222>  (35)..(36)
<223>  Nukleotid 35 und 36 sind verbunden über einen HEG-Linker

<400>  6
ctacagaact cagacaagat gtgaactaca atgttgctca tactacaatg tcacttactg      60

taagagcaga                                                              70


<210>  7
<211>  60
<212>  DNA
<213>  Artificial Sequence

<220>
<223>  Mismatch-Controller-Oligonukleotid



<220>
<221>  misc_feature
<222>  (28)..(46)
<223>  Das mittlere Segment des Oligonukleotids umfasst
       2´-O-Me-Nukleotid-Modifikationen

<220>
<221>  modified_base
<222>  (41)..(41)
<223>  Uracil instead of thymidine

<220>
<221>  misc_feature
<222>  (60)..(60)
<223>  3´-Phosphat-Gruppe zur Blockade einer möglichen Verlängerung
       durch Polymerase

<400>  7
gctctaatct gtaagagcag atccctggac aggcaaagaa tacaggtaga agcatcagag      60


<210>  8
<211>  120

<212>  DNA
<213>  Homo sapiens

<220>
<221>  misc_feature
<222>  (1)..(33)
<223>  Bindungsstelle für das Block-Oligonukleotid

<220>
<221>  misc_feature
<222>  (83)..(94)
<223>  Bindungssequenz für das erste Primer-Oligonukleotid

<400>  8
tgacgtggac atcatgagag acatcgcctc tgggctaata ggactacttc taatctgtaa      60

gagcagatcc ctggacaggc aaggaataca ggtattttgt ccttgaagta acctttcaga     120

<210>  9
<211>  60
<212>  DNA
<213>  Artificial Sequence

<220>
<223>  Block-Oligonukleotid

<220>
<221>  misc_feature
<222>  (1)..(12)
<223>  Sequenzsegment  1 bis 12 umfasst LNA-Modifikationen

<400>  9
ccagaggcga tgtatctcat gatgtccaca acactgtagt atggtcttgt taagcaaaaa      60

<210>  10
<211>  44
<212>  DNA
<213>  Artificial Sequence

<220>
<223>  Controller

<220>
<221>  modified_base
<222>  (1)..(1)
<223>  Uracil instead of thymidine

<220>
<221>  modified_base
<222>  (4)..(4)
<223>  Uracil instead of thymidine

<220>
<221>  modified_base
<222>  (6)..(6)
<223>  Uracil instead of thymidine

<220>

<221>  modified_base
<222>  (8)..(8)
<223>  Uracil instead of thymidine

<220>
<221>  modified_base
<222>  (18)..(18)
<223>  Uracil instead of thymidine

<220>
<221>  modified_base
<222>  (22)..(22)
<223>  Uracil instead of thymidine

<220>
<221>  modified_base
<222>  (37)..(37)
<223>  Uracil instead of thymidine

<400>  10
taatctgtaa gagcagatcc ctggacaggc aaggaataca ggta                        44


<210>  11
<211>  59
<212>  DNA
<213>  Homo sapiens

<400>  11
cttacagtaa gtgacattgt agtatgagcg atccctggac aggcaaggaa tacaggtaa        59


<210>  12
<211>  57
<212>  DNA
<213>  Homo sapiens

<400>  12
cttacagtaa gtgacattgt agtatgagcg atccctggac aggcaagaat acaggta          57


<210>  13
<211>  57
<212>  DNA
<213>  Homo sapiens

<400>  13
cttacagtaa gtgacattgt agtatgagcg atccctggac aggcaaaata caggtaa          57


<210>  14
<211>  59
<212>  DNA
<213>  Homo sapiens

<400>  14
cttacagtaa gtgacattgt agtatgagcg atccctggac aggcaaggaa taaaggtaa        59


<210>  15
<211>  59
<212>  DNA
<213>  Homo sapiens

<400>  15
cttacagtaa gtgacattgt agtatgagcg atccctggac aggcaaagaa tacaggtaa        59


<210>  16
<211>  58
<212>  DNA
<213>  Homo sapiens

<400>  16
cttacagtaa gtgacattgt agtatgagcg atccctggac aggcaagaat acaggtaa         58


<210>  17
<211>  58
<212>  DNA
<213>  Homo sapiens

<400>  17
cttacagtaa gtgacattgt agtatgagcg atccctggac agcaaggaat acaggtaa         58


<210>  18
<211>  47
<212>  DNA
<213>  Artificial Sequence

<220>
<223>  Primer für PCR


<220>
<221>  misc_feature
<222>  (36)..(47)
<223>  Segment 36 bis 47 dient als Primer

<400>  18
acgaagctcg caagaactca gagtgtgctc gacactacct gtattcc                     47


<210>  19
<211>  22
<212>  DNA
<213>  Artificial Sequence

<220>
<223>  Primer für PCR


<220>
<221>  misc_feature
<222>  (1)..(1)
<223>  Tetramethyl-Rhodamin am 5´-Ende

<220>
<221>  misc_feature
<222>  (1)..(10)
<223>  Segment 1 bis 10 umfasst 2´-O-Me-Modifikationen

<220>
<221>  modified_base
<222>  (2)..(2)

<223>  Uracil instead of thymidine

<220>
<221>  modified_base
<222>  (5)..(5)
<223>  Uracil instead of thymidine

<220>
<221>  modified_base
<222>  (8)..(8)
<223>  Uracil instead of thymidine

<220>
<221>  modified_base
<222>  (9)..(9)
<223>  Uracil instead of thymidine

<220>
<221>  misc_feature
<222>  (10)..(11)
<223>  Nukleotid 10 und 11 sind über einen C3-Linker verbunden

<220>
<221>  misc_feature
<222>  (11)..(22)
<223>  Segment 11 bis 22 dient als Primer

<400>  19
ctgatgcttc tacctgtatt cc                                                22


<210>  20
<211>  56
<212>  DNA
<213>  Artificial Sequence

<220>
<223>  Controller-Oligonukleotid



<220>
<221>  misc_feature
<222>  (1)..(39)
<223>  Das Segment 1 bis 39 umfasst 2´-O-Me-Nukleotid-Modifikationen

<220>
<221>  modified_base
<222>  (1)..(1)
<223>  Uracil instead of thymidine

<220>
<221>  modified_base
<222>  (4)..(4)
<223>  Uracil instead of thymidine

<220>
<221>  modified_base
<222>  (6)..(6)
<223>  Uracil instead of thymidine

<220>
<221>  modified_base
<222>  (8)..(8)

<223>  Uracil instead of thymidine

<220>
<221>  modified_base
<222>  (18)..(18)
<223>  Uracil instead of thymidine

<220>
<221>  modified_base
<222>  (22)..(22)
<223>  Uracil instead of thymidine

<220>
<221>  modified_base
<222>  (37)..(37)
<223>  Uracil instead of thymidine

<220>
<221>  misc_feature
<222>  (56)..(56)
<223>  Phosphat-Gruppe zur Blockade einer möglichen Verlängerung durch
      Polymerase

<400>  20
taatctgtaa gagcagatcc ctggacaggc aaggaataca ggtagaagca tcagag          56


<210>  21
<211>  48
<212>  DNA
<213>  Artificial Sequence

<220>
<223>  Fluoreszenz-Sonde


<220>
<221>  misc_feature
<222>  (14)..(15)
<223>  Nukleotid 14 und 15 sind verbunden über einen HEG-Linker

<220>
<221>  misc_feature
<222>  (22)..(23)
<223>  Nukleotid 22 und 23 sind verbunden über dT-BHQ1

<400>  21
tagtatgagc ttttgctcat acacaatgtc acttactgta agagcaga                  48